# EUROPEAN PATENT APPLICATION

(11) **EP 4 477 145 A1**
(43) Date of publication of application: **18.12.2024**
(21) Application number: 24181763.4
(22) Date of filing: 12.06.2024
(51) Int. Cl.: A61B 5/11, A61B 5/16, A61B 5/369, A61B 5/389, A61B 5/00, G16H 40/60

(54) **MOVEMENT BASED LEARNING CYCLE FOR ACCELERATED FUNCTIONAL RECOVERY FROM PHYSICAL, MENTAL, COGNITIVE AND EMOTIONAL DISORDERS**

(30) Priority: 12.06.2023 US 202363472350 P
(71) Applicant: Synphne Pte Ltd., Singapore 658071 (SG)
(72) Inventor: Banerji, Subhasis, 650222 Singapore (SG)
(74) Representative: Kompter, Hans-Michael

(57) **Abstract**

Disclosed is a learning model employed to treat patients with functional impairments due to psychological, mental cognitive or physical disorders or disabilities. The treatment includes accessing at which transition stage of the learning model that a subject has movement transition deficits based on the subject's bio-signals, such as brain state and muscle state signals. The movement transition deficits are addressed so that the subject can attain an upward spiral, accelerating functional recovery or development and avoid a downward spiral towards compensation and slowing down of recovery.

## Description

### CROSS-REFERENCE TO RELATED APPLICATION

This application claims priority to US Provisional Patent Application filed on Ser. No. 63/472,350 filed on June 12, 2023. This application also cross-references to co-pending US Patent Application Ser. No. 18/242,538 filed on September 6, 2023 which is a continuation of US Patent Application Ser. No. 15/768,556 filed on April 15, 2018. All disclosures are herein incorporated by reference for all purposes.

### FIELD OF THE INVENTION

The present disclosure generally relates to treating patients with psychological, mental, cognitive or physical disorders or disabilities, such as after a stroke or a brain injury. The treatment can also apply to patients with developmental delay, acute stress disorders, including emotional paralysis after stress disorders, mild cognitive impairments (MCI) as well as similar types of disorders or impairments. In particular, the treatment can be for any form of dysfunction or disorder. The treatment is based on a learning model to change how we transition through different stages of emergent movement using the patient's bio-signals, such as brain and muscle signals, which represent brain and body states and their transitions over a particular period in time.

### BACKGROUND

Treating or rehabilitating a patient with a functional impairment, such as that resulting from a stroke, or a brain injury as well as psychological, mental, cognitive or physical disorders or disabilities, development delay, acute stress disorders, mild cognitive impairments (MCI) and similar types of disorders or impairments, typically involves repetitively performing tasks related to the functional impairment. For example, for a patient suffering from partial paralysis of a limb, such as an arm, rehabilitation includes repetitively moving the arm. This also includes, for example, rehabilitating patients suffering emotional paralysis from stress disorders.

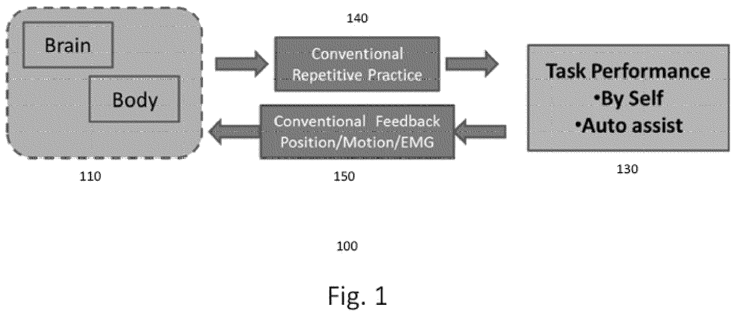

Fig. 1 shows a conventional rehabilitation model 100 used in treating patients with functional impairments. As shown, a patient 110 performs a task 130. The task, for example, may depend on the functional impairment. The patient repetitively performs the task 140. Feedback 150 is provided to the patient. The feedback is at a gross or obvious level (motion, position, trajectory, velocity) to enhance the capability of the patient to self-correct the next repetition.

Conventional rehabilitation models, as discussed, do not evaluate feedback based on body and mind states. As discussed, feedback for conventional rehabilitation models only considers physical motion at a gross or obvious level, including position, trajectory and velocity.

We have discovered that the mind and body, including the heart, have a positive effect on movement and neuroplasticity. The present disclosure relates to treating patients with neurological, emotional, movement or mental disorders by using a movement paradigm as a learning model to leverage neuroplasticity.

### SUMMARY

Effective treatment for accelerated recovery of patients with neurological damage or dysfunction is disclosed. In one embodiment, the present disclosure is related to a method for accessing and treating a subject with functional impairment or disorder. The method includes providing a movement-based learning model having multiple stages and transitions between stages. The method also includes fitting the subject with sensors for measuring brain state signals and muscle state signals. The method also includes performing a relaxation process prior to a treatment session. The relaxation process generates a baseline brain state and a baseline muscle state which indicates that the subject is in a relaxed focus state. The method further includes displaying a video of a functional exercise on a user interface (UI) for recovering a function of the functional impairment for the subject to perform. Displaying the exercise includes various modes including speeding up the video so the subject performs the functional exercise faster, slowing down the video so the subject performs the function exercise slower and pausing the video to allow the subject to rest. The various modes depend on a current brain state and a current muscle state to ensure the subject maintains an upward spiral in the transition between the stages of the learning model to accelerate recovery of the movement function.

In another embodiment, the present disclosure is related to a system for assessing and treating a subject with functional impairment or disorder. The system includes a processor unit. The processor unit includes a memory storing a treatment program for assessing and treating the subject, the program includes a movement-based learning model having multiple stages and transitions between stages. The processor unit also includes a processor. The processor is configured to run the treatment program during a treatment session for the subject. The system also includes a display unit. The display unit includes a user interface for displaying a video instruction from the treatment program for the subject to perform during the treatment session. The system further includes a sensor unit. The sensor unit includes sensors for measuring the bio-signals of the subject during the treatment session. The processor is configured to display the video instruction for the subject to perform to recover a function of the functional impairment, the processor is configured to display the video instruction in different modes based on the bio-signals of the subject. The different modes include a fast mode for the subject to perform the functional exercise faster, a slow mode for the subject to perform the functional exercise slower and a pause mode for the subject to pause performing the functional exercise. The different modes are run based on the bio-signals of the subject to ensure that the subject maintains an upward spiral in the learning model during the treatment session to accelerate recovery of the movement function.

These and other advantages and features of the embodiments herein disclosed, will become apparent through reference to the following description and the accompanying drawings. Furthermore, it is to be understood that the features of the various embodiments described herein are not mutually exclusive and can exist in various combinations and permutations.

### BRIEF DESCRIPTION OF THE DRAWINGS

In the drawings, like reference characters generally refer to the same parts throughout the different views. Also, the drawings are not necessarily drawn to scale, with emphasis instead generally being placed upon illustrating the principles of the invention. In the following description, various embodiments of the present invention are described with reference to the following drawings, in which:
Fig. 1 illustrates a conventional rehabilitation model for a patient recovering from a functional impairment;
Fig. 2 illustrates a cycle representing the genesis and nature of movement;
Fig. 3 illustrates a learning or neuroplasticity cycle;
Fig. 4a illustrates a learning cycle with adjacent stages stuck in loops;
Fig. 4b illustrates movement transition deficits due to disease and disorders in the learning cycle;
Fig. 4c illustrates an embodiment of metrics for measuring deficits in movement in the transition stages of the learning cycle;
Fig. 5 shows an embodiment of a learning model;
Fig. 6 shows an embodiment of an adaptive system for rehabilitating a patient after mental illness or disability due to neurological conditions and neurological dysregulation;
Fig. 7 shows an embodiment of a model integrated into an adaptive system for treating patients with neurological damage to accelerate functional recovery or development; and
Figs. 8a-8e illustrate transitions from one stage to another in the model.

### DETAILED DESCRIPTION

Embodiments relate to treating patients with neurological damage to accelerate recovery by using a learning model based on neuroplasticity to engage movement to restore function of the impairment. The functional impairment may be due to neurological conditions and neurological dysregulation after a mental illness, such as a stroke, or disability.

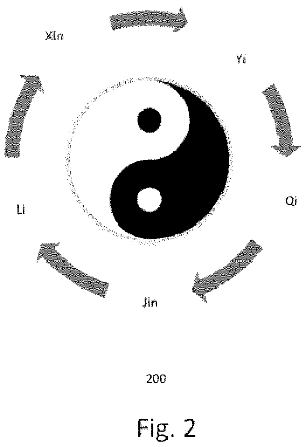

To restore movement, it is important to first understand the genesis and nature of movement. Fig. 2 illustrates a cycle 200 representing the genesis and nature of movement. The cycle includes Xin, Yi, Qi, Jin and Li stages. Xin corresponds to the heart/mind, such as desire or a thought, Yi corresponds to intent or goal, Qi corresponds to energy, Jin corresponds to planning and conceptualization and Li corresponds to muscles or groups of muscles.

Movement first emerges in its emotional form as a desire or a thought through Xin. The desire leads to the intent or goal to cure the desire through Yi. Intent directs energy through Qi. This energy may be aggressive, defensive or neutral. Energy triggers the planning and conceptualization of appropriate action or force through Jin. In other words, Qi supports Jin. As illustrated, the movement cycle transforms emotion to muscle activity in order to achieve the desired goal.

As an example, the movement cycle related to hunger is described. First, the emotional form of movement or Xin is the desire or the thought of Hunger. Xin leads to the intent or goal to cure the hunger through Yi. For example, the goal may be to walk to the fridge to get food. Yi directs or recruits energy to apply the intent to cure the hunger through Qi. For example, Qi is directed by Yi. Jin triggers the planning and conceptualization needed to get the food. For example, Qi supports Jin. Qi together with Jin activates muscles or groups of muscles needed to get the food and to eat the food through Li. For example, Qi and Jin support Li.

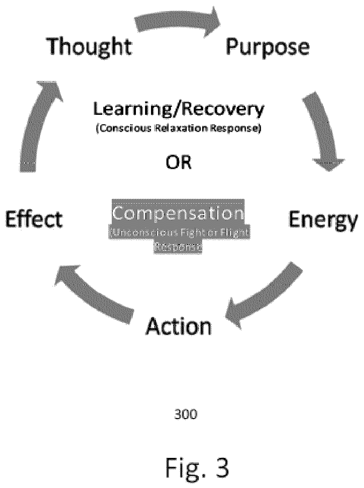

It is also important to understand how learning through neuroplasticity occurs. Fig. 3 illustrates a learning or neuroplasticity cycle 300. As shown, the neuroplasticity cycle includes 5 stages, similar to the movement cycle. The stages of the neuroplasticity cycle include thought, purpose, energy, action and effect. Thought or intent is neuroplasticity in emotional form. Recovery or compensation emerges as soon as thought starts crystallizing to purpose. Energy supports the purpose while effect is supported by energy and action.

Neuroplastic change is a constant mix of good adaptation (learning) and maladaptation (compensation). Similarly, movement is a constant mix of aggression and pacifism. Effective neuroplastic change depends on self-regulation of good adaptation (recovery and learning). Similarly, effective and appropriate movement depends on conscious self-regulation. Both movement and neuroplasticity are initiated by intent (volition) at its root. The nature of intent or volition is emotion. As such, the achievement of neuroplastic change is effectively mediated through movement. For example, when a sea urchin attaches itself to a rock in adult life and stops moving, its nervous system wastes away through atrophy. Consequently, all forms of neuroplastic change hinge on the conscious self-regulation of movement or the need for future movement.

As illustrated, movement originates in cognition as intent driven by desire or thought. The articulation and reinforcement of intent is therefore an important first step in the restoration of movement. To access and treat the loss of movement, it is necessary to simultaneously address the restoration of holding intent or cognition, including short-term memory.

As for the neuroplasticity cycle, it indicates the stage-wise execution of the generation of movement with the self-regulation necessary at each stage of the thought-purpose-energy-action-effect cycle. Movement and learning can be achieved in two ways. The first way is through unconscious, reflexive compensation. The second way is through mindful recovery and learning.

Regarding the first way, the modulation of thought is feedback in nature. For example, thought is modulated by effect. We discovered that with the feedback modulation, the cycle is generally finite and stops quickly in those with impairments and dysfunction due to exhaustion or pain. In most instances, the outcome is a failure. This is referred to as a downward spiral. A downward spiral occurs when the focus is on success and failure in the movement being attempted, which is an effect-focused approach.

For the second way, the modulation is feedforward. For example, thought is modulated by purpose. When this happens, the spiral is sustainable and may be infinite. This is conversely referred to as an upward spiral. The occurrence of an upward spiral takes place when attention is sustained on purpose. Although success and failure are considered, they are only considered to the extent of how and what to self-regulate better in the next attempt of the movement.

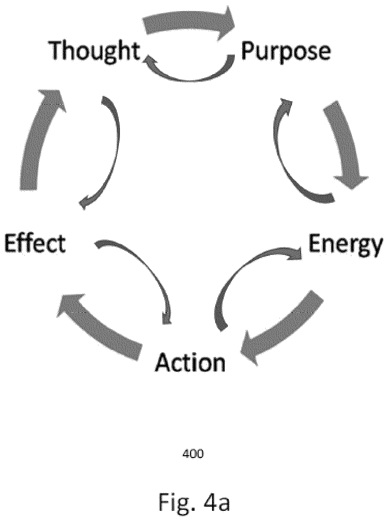

Moreover, we have discovered if learning is not properly supported, the learning cycle can be stuck in small or smaller movement loops. For example, without proper feedforward support in the stages of the learning cycle, an upward spiral does not occur and learning is unsustainable. Fig. 4a illustrates a learning cycle with adjacent stages stuck in loops. For example, thought and purpose can be stuck in a small loop as in the case when the purpose lacks clarity. Energy and purpose can be stuck in a small loop when energy is low, such as when the patient is exhausted, in pain or procrastinating. As for action and energy, they can be stuck in a small loop if energy is unavailable to complete the action due to exhaustion, pain or loss of purpose or distraction of intent. Action and effect can be stuck in a small loop. For example, unsuccessful action due to exhaustion leads to anger and frustration. Thought and effect can also be stuck in a small loop. In the case of continued unsuccessful effects, debilitating thoughts or depression can set in. In other words, the learning cycle does not go through all the stages of the cycle or spiral in sequence to result in an upward spiral. In such cases, learning and the leveraging of neuroplasticity are not sustainable.

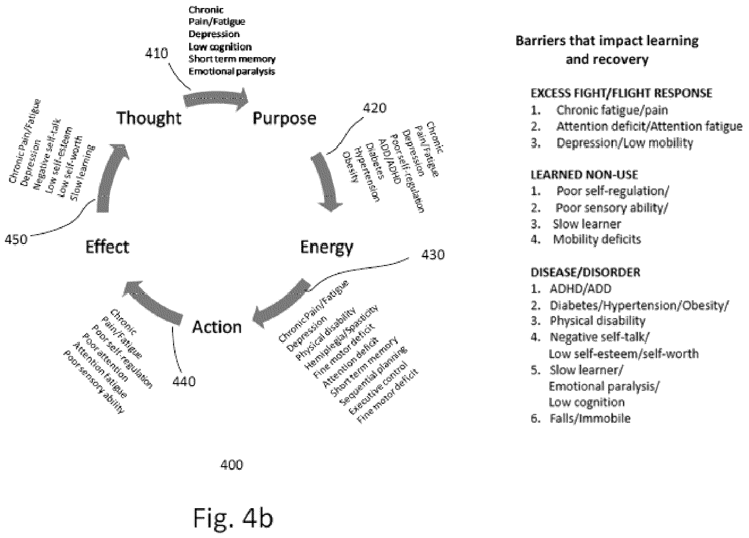

Fig. 4b illustrates movement transition deficits due to disease and disorders in the learning cycle 400. As previously discussed, it is important that an upward spiral is maintained in the learning cycle. As illustrated, movement transition deficits can occur from one stage to the next stage. For example, deficits can occur with transition 410 from thought to purpose, transition 420 from purpose to energy, transition 430 from energy to action, transition 440 from action to effect and/or transition 450 from effect to thought. The transition deficits can occur due to disease or disorder.

Transitions in movement elements become difficult if certain clinical symptoms are present. Disorders, dysfunctions and symptoms which makes the transitions difficult are listed for each of the transitions. For example, depression may impede the ability to transition from thought (intent) to purpose. This in turn impedes the relearning of movement as a whole. If any of these symptoms are making a transition difficult, a therapist may assist physically in the transition through instructions, nudges or physical assistance such as passive mobilization. Assisting in this manner but only focusing on one transition at a time is recommended. Failures at each of these stages on the part of the patient are expected at the initial stages of the relearning movement.

Also listed in Fig. 4b are factors that manifest as barriers that impact learning and recovery. As shown, the first factor includes uncontrolled or exaggerated fight or flight response. The characteristics of this factor typically include chronic fatigue, pain, attention deficit, attention fatigue, depression and low mobility. The second factor is a compensated use of the brain and muscle, where certain areas and muscles waste away due to non-use, impacting the ability to leverage them in the recovery and learning process. The third factor is disorders and dysfunctions may be embedded in the patient. Such disorders and dysfunctions may include ADHD, ADD, diabetes, hypertension, obesity, physical disability, negative self-talk, low self-esteem, low self-worth, slow learning, emotional paralysis, low cognition and immobility due to injuries, such as a fall.

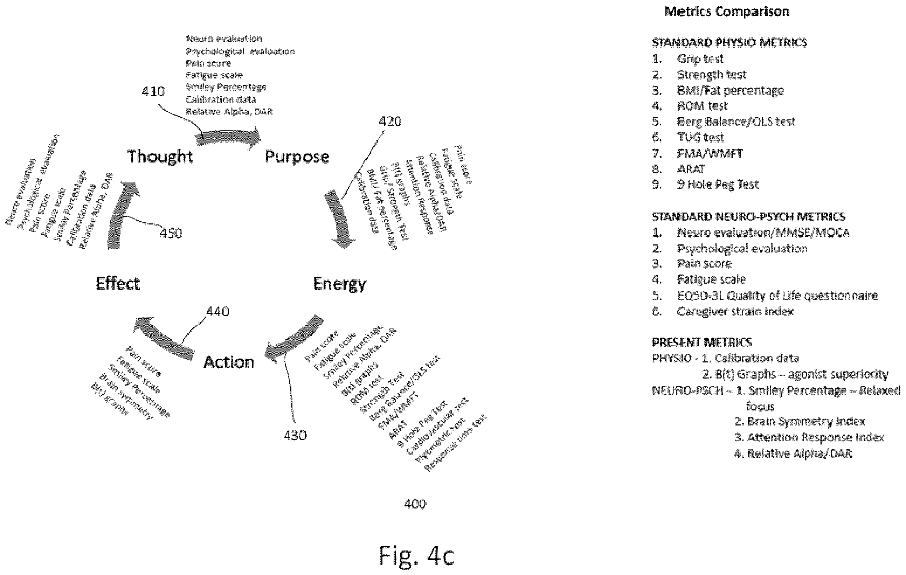

We have developed metrics to measure deficits in movement in the transition stages. Fig. 4c illustrates an embodiment of metrics for measuring deficits in movement in the transition stages of the learning cycle 400. As shown, a present embodiment of metrics may include physiological and neuro-psychological metrics. The physiological and neuro-psychological metrics may be referred to as brain-muscle metrics as measured by the system. The present metrics are bio-signal based. For example, the brain-muscle metrics are extracted from EEG and EMG signals as well as other data.

In one embodiment, the physiological metrics include calibration data and B(t) graphs. The calibration data may be derived from, for example, a baseline session at the beginning of a treatment session. The baseline session may include a relaxation session and calibration movements to obtain baseline data in preparation of the treatment session. The baseline session, for example, may include a 60 second relaxation session followed by a movement session, such as moving the forearm, wrist, finger and thumb movements to record data over 3 repetitions and build a baseline for the session. The relaxation session is performed prior to the commencement of the treatment session. However, the movement session need not be performed prior to every treatment session. For example, the movement session may be performed for muscle calibration every 3 to 6 treatment sessions, depending on the type of patient. As for the B(t) graphs, they measure the body stage at time t, which is the balance between agonist and antagonist muscles. The B(t) graphs are based on EMG signals.

As for the neuro-psychological metrics, they include a smiley percentage, a brain symmetry index, an attention response index, a relative alpha measure and a delta-to-alpha ratio (DAR). The smiley percentage is the percentage of time during an activity, exercise or session that the patient is relaxed and focused. The smiley percentage is based on the mind state at time t M(t), which is the state of arousal based on EEG signals. The attention response index measures the time it takes for the relaxed focus to disappear after a patient starts to perform a task. The relative alpha measure indicates states of improved relaxation while DAR is a proxy marker for attention. DAR is used instead of beta waves to avoid inducing stress. In addition, the range of motion (ROM) of various joints may be manually measured. The ROM of the various joints may be part of the physiological metrics.

This scoring at each transition enables assessing and measuring the progress of the patient regarding learning and recovery. The ranking of the transitions based on transition scores is employed to prioritize which transition needs to be treated first. In one embodiment, upstream transitions closer to thought will take precedence over downstream transitions closer to effect.

The brain and the body are thus inseparably linked. Both the brain and body contribute significantly to neuroplasticity to improve recovery of function in a neurologically damaged patient. With this understanding, we have developed a learning model focused on maintaining an upward spiral for motor function recovery using feedback and feedforward. The learning model is based on bio-signals and a user interface on a display to determine if a patient is stuck in smaller loops of the learning cycle.

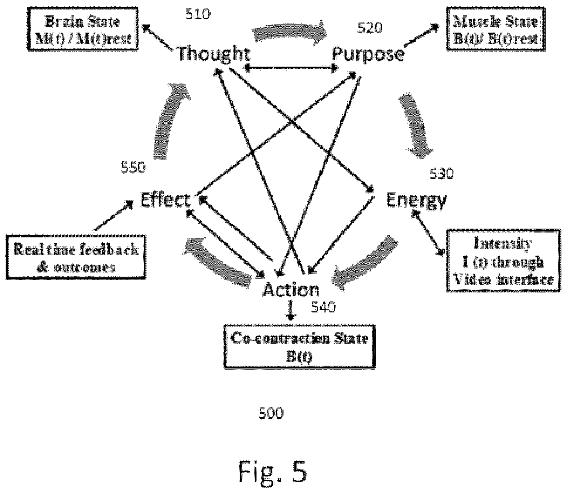

Fig. 5 shows an embodiment of a learning model 500. The model employs self-regulation. The model includes a learning cycle with a thought stage 510, a purpose stage 520, an energy stage 530, an action stage 540 and an effect stage 540. Bio-signals are taken from a patient during a treatment session. The bio-signals include mind or brain state signals, and body or muscle state signals. Brain state signals are EEG signals while muscle state signals are EMG signals.

As can be seen, the brain state modulates the intensity of therapy directly (thought to energy). For example, during a session, a video is played for the patient to watch and to perform exercises as shown. The brain state of the patient impacts the speed at which the video plays. For example, if the brain state indicates a relaxed focus state for more than a pre-set time continuously, the video speeds up or runs faster. This is because the patient is in a relaxed and focused state, which is conducive to learning or performing the task successfully. The duration of the pre-set time may be about 60 - 90 seconds, depending on the type of patient. Other durations for the pre-set time may also be useful. In some cases, the video may speed up for the next task after a task has been completed successfully. Conversely, if the brain state indicates the patient is not in a relaxed and focused state, the video switches back to a slow speed.

In one embodiment, the time it takes to perform a task may depend on the type of task. For example, a wrist extension task may normally take about 1-2 seconds to perform while a task for pouring water into a glass may take about 15 seconds. The normal speed of the task can be used as a reference for playing the video. In one embodiment, the system is configured to play the video at three different speeds. For example, the video can be configured to play at a slow speed, a normal speed or a fast speed. Providing other numbers of speed settings at which the video can play may also be useful. In one embodiment, slow speed may be about 5 times slower than the normal speed while fast speed may be about 2 times faster than the normal speed. Other ratios for fast and slow speed relative to normal speed may also be useful. To begin, the video may play at the normal speed. The video can be sped up and slowed down based on the brain state of the patient.

In one embodiment, a patent's relaxed and focused state is based on a baseline brain calibrated at the beginning of the session using measured EEG signals. For example, the patient may prepare himself/herself to be in a relaxed and focused state at the beginning of the session. The EEG measured when the patient is in a relaxed and focused state serves as the baseline EEG measure. In one embodiment, a patient may be considered to not be in a relaxed and focused state when the current EEG measure is 20% of the baseline EEG measure. Other percentages may also be used to indicate when a patient is or is not in a relaxed and focused state. For example, the range may be varied based on the severity of the patient's condition. In the case of a patient with slow cognition, a wider boundary may be applied.

We have correlated that when the patient is in a relaxed and focused state, the patient is in an upward spiral. This puts the patient in a conducive state to learn through neuroplasticity. Speeding up the repetitions while maintaining a stable relaxed focus state enables the patient to accelerate functional or movement recovery through neuroplasticity.

On the other hand, when the patient is not in a relaxed and focused state, the patient is in a downward spiral. This is a state which is not conducive to learning through neuroplasticity. In such instances, the video may switch to a slow speed to slow down the patient's performance of the task. This may enable the patient to recover, for example, from exhaustion or frustration and return to a relaxed focus state. Once the patient has recovered and is back to being in a focused and aware state, the video speeds up to impart learning through neuroplasticity to accelerate functional recovery. In other instances, the task can be switched to a simpler task in which the patient is able to perform while maintaining a relaxed focus state.

As for muscle state, it directly influences action by modulating agonist-antagonist balance (or co-contraction state or hyperactivity state). This balance changes based on whether the task is merely a range-of-motion task such as a joint extension or flexion (low co-contraction required) or a functional task such as gripping a pen or bottle (higher co-contraction required). As such, the EEG signals supply the feedforward whereas the EMG signals, which correlate with the initialization of muscle state, supply the feedback in the automated cycle.

Real-time feedback, feedforward and control can be achieved when EEG signals are combined with EMG signals to sense muscle activation and de-activation in a time-locked manner. This provides an opportunity for the therapist and patient to not only work with the body but also the mind during the rehabilitation. This ensures that the patient is not stuck in smaller loops and maintains an upward spiral. For example, when the therapist notices that the patient is exhausted and not performing the tasks successfully, the therapist may pause the video to enable the patient to recover and refocus. During the pause, the patient may focus on breathing based on the breathing guide to allow the patient to return to the appropriate brain state (relaxed and focused). Alternatively, the therapist may initiate a relaxation video to play on the UI to relax and put the patient back in the appropriate brain state.

In the case where no therapist is present, the system may analyze the brain and muscle signals and determine that the patient is not in a relaxed and focused state. In such instances, the system may set off an alarm to alert the patient when his brain state is not in the appropriate state (e.g., the patient is stuck in a smaller loop). The system may pause the video to stop the patient from further performing the task until the patient's brain state returns to the appropriate state. For example, the patient may perform breathing according to the breathing guide to facilitate the patient returning to the appropriate brain state. In addition, the system may play a relaxation video to relax the patient. This helps to return the patient to a relaxed and alert state, conducive to learning supported by neuroplasticity.

Engaging the mind and body together using appropriate and quantified feedback and feedforward parameters will enhance the conditions for incremental associative learning and muscle recovery. This will in turn kick-start or accelerate the rehabilitation process. We have discovered that under such conditions, patients may achieve systemic gains in functional performance.

A bio-signal based index or measure that signifies the ability to synergize a mind-body way of training while doing therapy may be employed. The bio-signal based index is based on M(t) and B(t). The Bio-signal based index is described in US Patent Application Ser. No. 16/851,080 filed on April 16, 2020, which is herein incorporated by reference for all purposes. Such an index could also serve as an indicator for future recovery which is based on the patient's ability to self-regulate and learn rather than merely on the existing impairment level or some other historical parameter. This is important for patient empowerment and formulation of goals.

In one embodiment, the system employs first and second primary indices. The first primary index may be a smiley or graphical index, which may include a smiley face. The smiley index is based on the brain signal M(t). The smiley index indicates when the patient's brain wave is in a sustained relaxed and focused state. For example, a smiley face is displayed on the UI to show that the patient is in a sustained relaxed and focused state. The second primary index is based on the muscle signal B(t). For example, the B(t) graph may be used to indicate when the patient possesses synergistic muscle control.

Furthermore, the learning cycle is used to assess or diagnose the patient. For example, the learning cycle can serve as a heat map, indicating at which transition of the stages of movement (thought-purpose-energy-action-effect) the patient is doing well or doing poorly. Once the diagnosis is made, a customized treatment plan can be devised to accelerate recovery or advancement of function. For example, the treatment can be customized or tailored for those specific transitions. The focus of the treatment plan may be configured to address the worst to least worst transitions in sequence. Other configurations of treatment plans may also be useful. This results in personalized or customized treatment plans for each individual to accelerate recovery based on identified deficiencies or weaknesses in the stages of the learning cycle. For example, for two patients who frequently drop things, one may be due to a weak grip while the other may be due to a lack of focus. The one with the weak grip will assigned to perform grip strength exercises while the one with the lack of focus will be assigned to perform attention-related exercises.

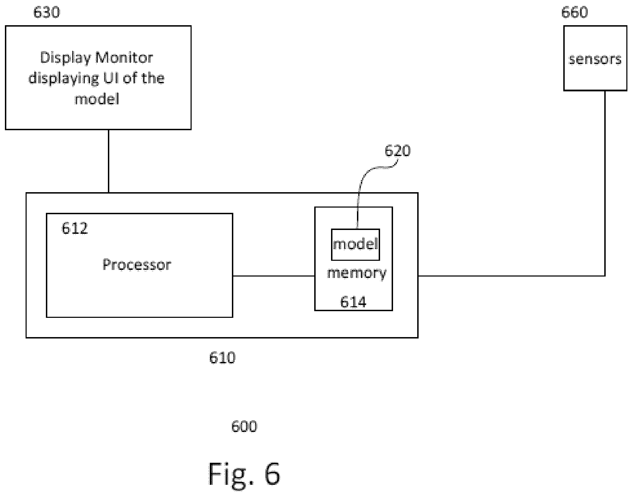

Fig. 6 shows an embodiment of an adaptive system 600 for rehabilitating a patient after mental illness or disability due to neurological conditions and neurological dysregulation. As shown, the system includes a processing unit 610, a display unit 630 and a sensor unit 660. The processing unit, for example, may be a general computer having a processor 612 and a memory 614. The processor is configured to run a rehabilitation program or developmental program 620 stored in the memory.

The sensor unit, in one embodiment, includes EEG sensors and EMG sensors for receiving EEG and EMG signals from the patient. For example, during a treatment session, the patient is fitted with sensors to transmit EEG and EMG signals to the processing unit. For example, the sensors transmit the signals to the processor. Sensors for measuring other bio-signals, such as heart signals, may also be useful. Transmission of the signals may be transmitted wired or wirelessly, depending on the configuration of the sensors.

The treatment application, in one embodiment, includes the learning model. The learning model, for example, is described in Fig. 4. In addition, the memory may include different functional exercises for the patient to perform. The exercise selected is displayed on a user interface of the program on the display unit for the patient to perform. The exercise selected for the patient to perform may depend on the function or movement for recovery. For example, the exercise may be based on the movement or activity that needs to be achieved and broken down into its components. Other factors may include the severity and tolerance of the patient, such as performing an easy task versus a difficult task. This can be based on the smiley face, brain symmetry and the B(t) graph.

Treatment may include different types of exercises based on mind state and body state measures, such as those described in US Patent Application Ser. No. 16/851,080 filed on April 16, 2020, which is herein incorporated by reference for all purposes. Treatment may also include different types of exercises based on mind state, body state and emotional state measures, as described in US Patent Application Ser. No. 18/242,538 filed on September 6, 2023, which is also herein incorporated by reference for all purposes.

In addition, the treatment may be based on bio-signals to ensure that the patient or subject is not stuck in small loops of the learning model. The treatment may also be based on diagnosis which identifies which stage of the transitions of the learning model in which the subject has deficiencies. For example, the diagnosis may serve as a heat map for treating the patient to ensure accelerated recovery of function or accelerated development.

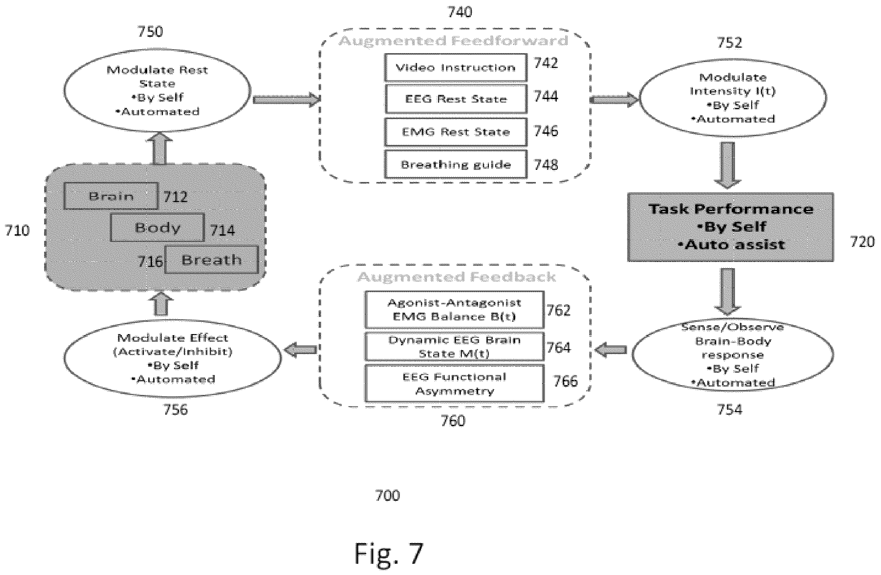

Fig. 7 shows an embodiment of a model integrated into an adaptive system 700 for treating patients or subjects with neurological damage to accelerate functional recovery. The system, for example, may be integrated with a human-computer interface so that learning cycles can be personalized not just patient-wise, but also session-wise. This system is advantageous in, for example, stroke rehabilitation because deficits in cognition, muscle tone and endurance are transient and may vary significantly from one session to the next for the same patient, thus affecting performance.

In one embodiment, the model includes an augmented feedforward (AFF) module 740 and an augmented feedback (AFB) module 760. The AFF module includes video instruction 742, EEG rest state measures 744, EMG rest state measures 746 and a breathing guide 748.

EEG and EMG rest state measures are measured at the beginning of each treatment session. For example, to prepare for a treatment session, the patient should be in a relaxed state. The patient, for example, in preparation, wears the EEG and EMG sensors for measuring the EEG and EMG signals. The patient should be as relaxed as possible, but still in an aware state. For example, the patient should be motionless and breathing freely while maintaining awareness. The EEG and EMG measured while in this state will be the EEG and EMG rest state measures.

To prepare to be in a relaxed and aware state, the subject should breathe freely and in a relaxed manner without any effort exerted. The mouth can be opened to allow the whole upper body to breathe freely. Also, feeling sleepy or yawning may facilitate the subject to be in a relaxed and aware state. This relaxed state may cause the feeling of being in a low-energy state. This is acceptable as long as the subject maintains awareness. Additionally, the subject may lie down for a few minutes on their back or sides. Afterward, the subject may sit in a chair and sequentially relax the face, the scalp, the neck, the shoulders, the chest, the stomach and the lower back all while maintaining awareness of the skin on the face, soles of the feet and fingertips. Once prepared, the subject should remain seated in the chair for the session. The subject should position themselves as far back into the chair seat as possible, ensuring that their feet are flat on the floor, knees flexed at approximately 90 degrees and breathing freely with the mouth slightly open. It is important to maintain a state of awareness and relaxation until the session begins. Other procedures to put the subject in a relaxed and aware state may also be useful.

The video instruction serves as a reference to facilitate goal-oriented learning. For example, the video instruction includes a task to be performed by the patient. The task is based on the function or movement to be rehabilitated. For example, if the function is to improve right arm movement, the task to be performed may be associated with using the right arm. In the case of improving the movement of fingers of the right hand, the task may be to pick up an object using the right hand. The breathing guide is a timed indicator bar which provides temporal guidance for inhalation time and exhalation time during task performance. For example, breathing is included as a learning area while performing a selected task. The video instruction and breathing guide may be displayed in a user interface (UI) on a display of the adaptive system. The video instruction may be played in a loop to induce repetition of the task. In some embodiments, the heart rate may also be measured to provide additional bio-information of the patient.

As for the AFB module, it includes muscle state signal B(t) and brain state signal M(t) measured during task performance 720 by the patient. For example, agonist-antagonist EMG balance 762, dynamic EEG brain state 764 and EEG functional asymmetry 766 are determined based on processed brain and muscle state signals.

During a session, the subject 710 performs a task 720. The performance of the task may be automatically assisted by the video instruction shown in the UI. The patient, for example, engages the brain, breath and muscles. The brain and muscle states are calculated from processed EEG and EMG data. These states represent the subject's homeostasis measures. These states are used to modulate attention, energy and mechanical movement respectively. The bio-signals, such as EEG and EMG signals are continuously monitored and analyzed. The bio-signals are continuously processed and analyzed in real-time. Of course, real-time may include inherent processing delays, which are negligible. The system may assist the subject when the subject is unable to regain or enhance the homeostasis measures on their own within a given time frame.

The bio-signals are used to control the treatment. For example, the brain (or thought) state modulates the intensity I(t) of therapy by controlling the speed at which the video demonstrates how fast the action should be performed. In addition, rest periods can be modulated automatically on the UI based on repetitive failure. Failure, for example, can be indicated by failure to regain a smiley face, for example, within 60 - 90 seconds, depending on the type of patient. Failure can also be detected by visual inspection, by the subject or by a therapist. In other cases, failure can be detected based on the use of AI. For example, the movement of the subject can be viewed by AI vision and compared to the video to determine success or failure. This changes the difficulty level of the therapy exercises, thus varying intensity in terms of the number of correctly performed repetitions per unit time. Furthermore, as recovery progresses, video instructions may not be needed. For example, the subject can automatically manage the intensity levels using the EEG homeostasis real-time measures as a monitoring tool.

The Muscle (or Purpose) state influences the strength applied by the muscles as well as the agonist-antagonist balance achieved in the performance of the action or task being performed. This performance of action, in some cases, may be assisted by an external device, such as an electrical stimulation device, a robot device or an exoskeleton device. For example, external devices may be used where the level of impairment of the patient makes it impossible to generate any movement without assistance.

Self-correction or self-regulation is a key to accelerated recovery or development of function. The model includes four stages of self-regulation. As shown, the stages of self-regulation are achieved by moderating rest 750, modulating intensity 752, visual observation of brain-body response 754 and modulating effect 756.

EEG and EMG signals are used to indicate the level of relaxation and readiness of the subject as the next feedforward in the form of the video (and/or audio) instruction. This helps in clarifying the purpose of the next immediate attempt at the movement. The first stage of self-regulation is to manage intensity and speed using the video instruction.

The second stage of self-regulation is based on the immediate response of the EEG and EMG signals based on the attempt at the movement using the bio-signals as feedback. Visual verification of the movement by the subject occurs nearly simultaneously, enabling real-time self-correction.

The result of the second stage of self-regulation is immediate and based on the visible outcome or effect of the movement, which is the third stage of self-regulation. This feedback can help to complete the outcome. For example, the outcome can be a success or failure. This may be corrected in the next repetition, for example, with better attention and muscle control.

The fourth stage of self-regulation is for the subject to return to the same state of relaxation and readiness at the start of the cycle. For example, rest can be modulated until the subject returns to the same state of readiness. The stages of self-regulation then repeat.

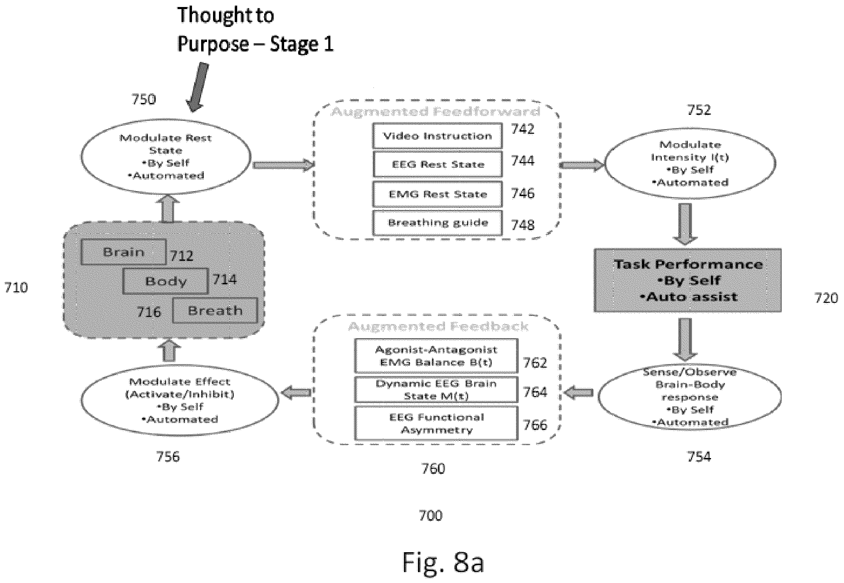

Figs. 8a-8e illustrate transitions from one stage to another in the model 700. The model of Figs. 8a-8e is similar to that of Fig. 7. Common elements may not be described or described in detail.

Referring to Fig. 8a, a first stage of the transition from the thought stage to purpose stage in the model 700 is illustrated. In the baseline transition of the first stage (from thought to purpose), the effect (the stage before thought) of the previous iteration or attempt should be considered or noted. For example, to achieve a baseline for the first stage transition to occur, the subject should be conscientious with an ability to slow down or pause. For example, the subject can practice pausing in life and work after an intense effort. The ability to momentarily experience stillness and not doing anything while holding an erect and relaxed physical posture (relaxed and not collapsed) and breathing freely can place the subject in a relaxed and aware state. Exercises can include allowing the body to breathe freely, including tracking the entry and exit of breath. In addition, video can be played to relax the subject along with the breathing guide, such as bears resting at the bottom of a tree.

The bio-signals can be analyzed to determine the shifts in the smiley index while simultaneously following the breathing bar of the breathing guide. Other relaxing exercises may also be useful. The relaxed and aware state reconfirms the purpose to initiate the next iteration, with first steps of many to fulfill the purpose, such as a specific task.

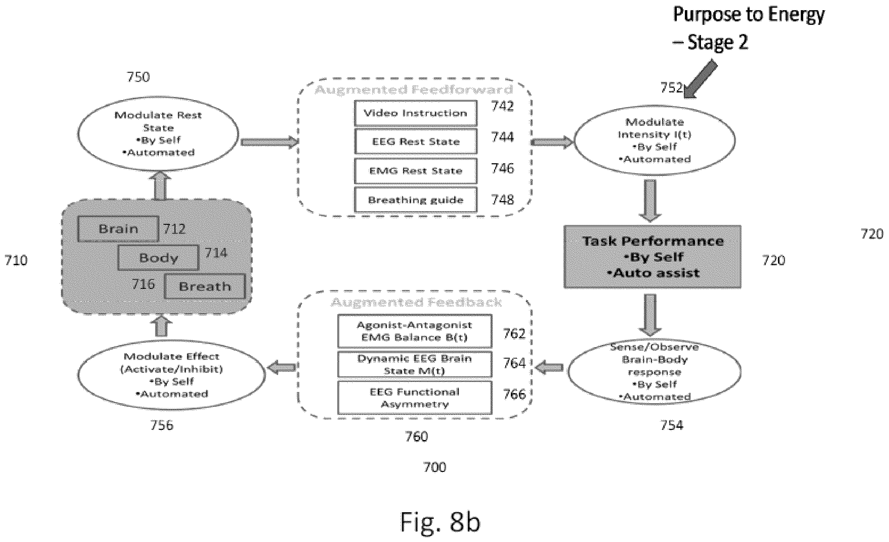

Fig. 8b illustrates a second stage of the transition from the purpose stage to the energy stage in the model 700. The transition requires recruiting energy appropriate to the demands of the task to be executed. The ability to maintain a high relative alpha, smiley and a relaxed body state B(t) (e.g., B(t) close to zero) just before transitioning to action is important.

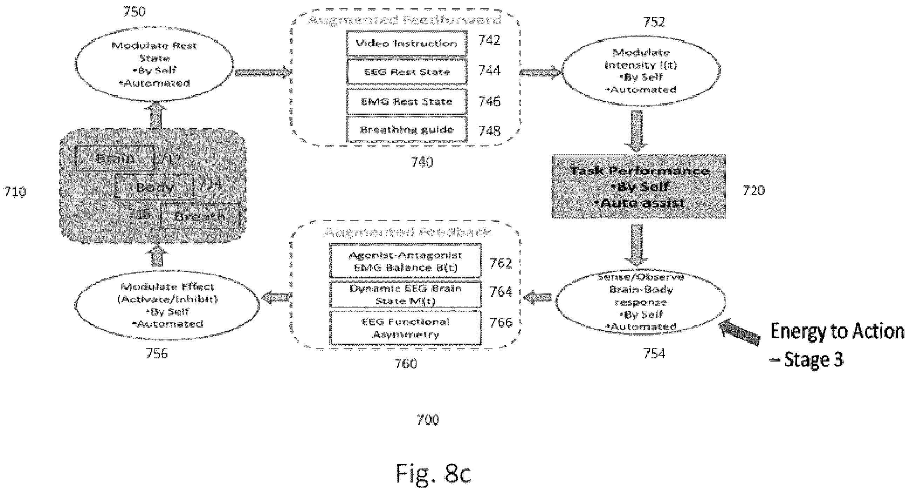

In Fig. 8c, a third stage of transition from the energy stage to the action stage in the model 700 is shown. In this transition, it is important to align the movement ROM as well as match the speed of movement with the video instruction. Furthermore, it is important to maintain a high relative alpha, smiley and a relaxed body state B(t) just before executing the action. This enhances the ability to self-correct to the extent possible by matching the speed of movement of the video instruction. The ability to maintain a relaxed state avoids a disruptive compensatory response.

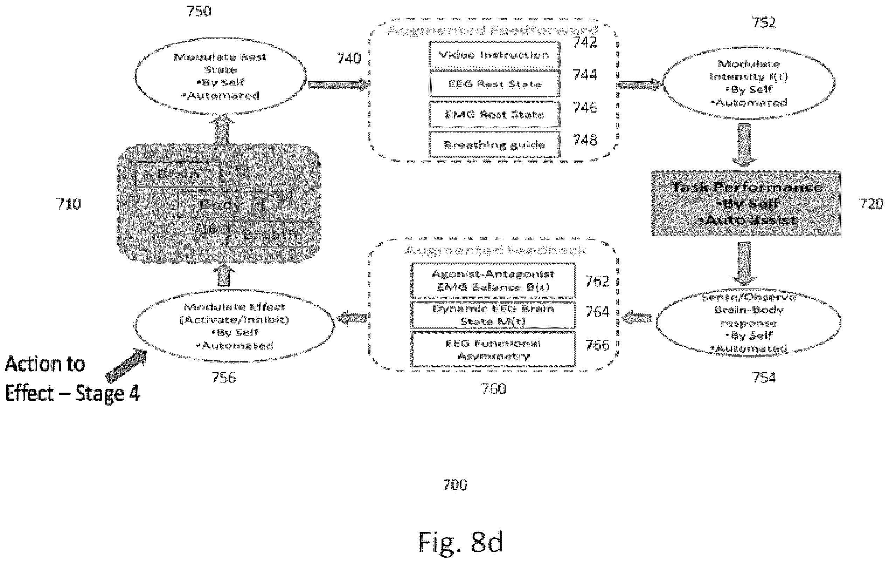

Referring to Fig. 8d, a fourth stage of transition from the action stage to the effect stage in the model 700 is illustrated. In this stage, it is important for the subject to complete the movement ROM shown in the video instruction and at the same instant as the video instruction. It is also important for the subject to maintain a relaxed state, such as a relaxed face, neck, shoulder and sternum along with a stable head and trunk. This facilitates the transition to the next stage.

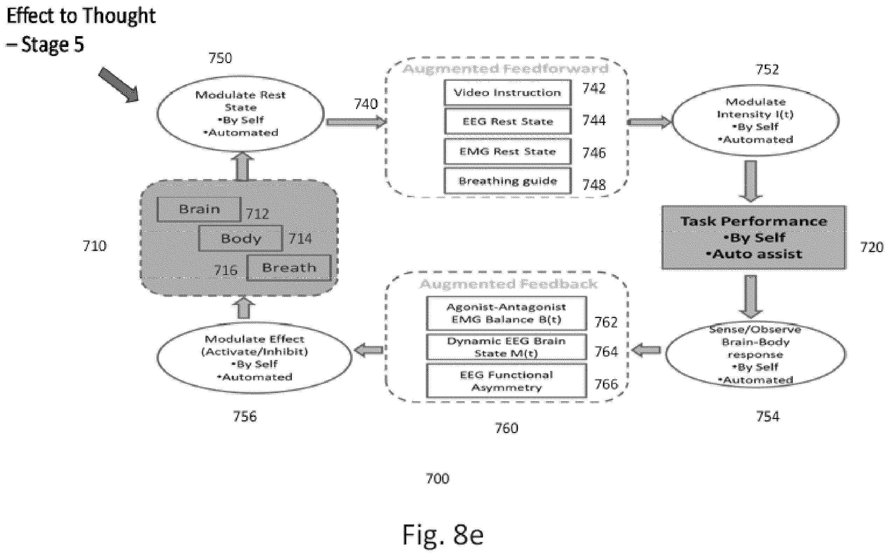

Fig. 8e illustrates a fifth stage of transition from the effect stage back to the thought stage in the model 700 is shown. To progress, it is important to mentally and emotionally downplay or be affected by the results (effect), whether good or bad. The subject should be able to relax and let go of the results in readiness for the next iteration/completion. For example, the subject should breathe fully to trigger the relaxation response instead of the habitual fight or flight response. This facilitates the transition to the next stage.

Being able to transition through the 5 stages of the model with an upward spiral achieves movement transition. This accelerates recovery or development of functions.

The present disclosure may be embodied in other specific forms without departing from the spirit or essential characteristics thereof. The foregoing embodiments, therefore, are to be considered in all respects illustrative rather than limiting the invention described herein. The scope of the invention is thus indicated by the appended claims, rather than by the foregoing description, and all changes that come within the meaning and range of equivalency of the claims are intended to be embraced therein.

## Claims

1. A method for assessing and treating a subject with functional impairment or disorder comprising:
providing a movement-based learning model having multiple stages and transitions between stages;
fitting the subject with sensors for measuring brain state signals and muscle state signals;
performing a relaxation process prior to a treatment session, wherein the relaxation process generates a baseline brain state and a baseline muscle state which indicates that the subject is in a relaxed focus state; and
displaying a video of a functional exercise on a user interface (UI) for recovering a function of the functional impairment for the subject to perform, wherein displaying the exercise includes various modes including
speeding up the video so the subject performs the functional exercise faster, slowing down the video so the subject performs the function exercise slower, pausing the video to allow the subject to rest, wherein the various modes depend on a current brain state and a current muscle state to ensure the subject maintains an upward spiral in the transition between the stages of the learning model to accelerate recovery of the movement function.

2. A system for assessing and treating a subject with functional impairment or disorder comprising:
a processor unit, the processor unit comprises
a memory storing a treatment program for assessing and treating the subject, the program includes a movement-based learning model having multiple stages and transitions between stages.
a processor, the processor is configured to run the treatment program during a treatment session for the subject;
a display unit, the display unit includes a user interface for displaying a video instruction from the treatment program for the subject to perform during the treatment session;
a sensor unit, the sensor unit comprises sensors for measuring the bio-signals of the subject during the treatment session; and
wherein the processor is configured to display the video instruction for the subject to perform to recover a function of the functional impairment, the processor is configured to display the video instruction in different modes based on the bio-signals of the subject, the different modes comprise
a fast mode for the subject to perform the functional exercise faster,
a slow mode for the subject to perform the functional exercise slower, and
a pause mode for the subject to pause performing the functional exercise, and
wherein the different modes are run based on the bio-signals of the subject to ensure that the subject maintains an upward spiral in the learning model during the treatment session to accelerate recovery of the movement function.
